Europäisches Patentamt

European Patent Office

Office européen des brevets

(19)

(11) Numéro de publication: **0 266 248**
**B1**

(12)

# FASCICULE DE BREVET EUROPEEN

(45) Date de publication du fascicule du brevet: **16.01.91**

(21) Numéro de dépôt: **87402227.0**

(22) Date de dépôt: **07.10.87**

(51) Int. Cl.⁵: **C 09 C 1/00,** C 09 C 3/08,
C 09 C 1/30, C 09 C 1/36

(54) **Pigments colorés à base d'un oxyde minéral du type silice, alumine ou oxyde de titane ou de zirconium et leurs procédés de préparation.**

(30) Priorité: **10.10.86 FR 8614103**

(43) Date de publication de la demande:
**04.05.88 Bulletin 88/18**

(45) Mention de la délivrance du brevet:
**16.01.91 Bulletin 91/03**

(84) Etats contractants désignés:
**AT BE CH DE ES FR GB GR IT LI LU NL SE**

(56) Documents cités:
**GB-A- 772 800**
**GB-A-2 117 783**

(73) Titulaire: **RHONE-POULENC CHIMIE**
**25, quai Paul Doumer**
**F-92408 Courbevoie Cédex (FR)**

(72) Inventeur: **Persello, Jacques**
**RN 83**
**F-01390 Saint André de Corcy (FR)**

(74) Mandataire: **Dubruc, Philippe et al**
**RHONE-POULENC INTERSERVICES Service**
**Brevets Chimie 25, quai Paul-Doumer**
**F-92408 Courbevoie Cédex (FR)**

Le dossier contient des informations techniques présentées postérieurement au dépôt de la demande et ne figurant pas dans le présent fascicule.

Courier Press, Leamington Spa, England.

EP 0 266 248 B1

# EP 0 266 248 B1

**Description**

La présente invention concerne des pigments colorés à base d'un oxyde minéral du type notamment silice, alumine ou oxyde de titane ou de zirconium et leurs procédés de préparation.

On sait qu'on utilise dans de nombreuses applications des charges minérales pour conférer à un matériau un ensemble de propriétés déterminées. Parmi les plus classiques de ces propriétés, on peut citer les propriétés optiques, mécaniques, rhéologiques en particulier.

Dans certaines applications, il est nécessaire d'employer ces charges en combinaison avec un pigment ou un colorant. Généralement ces compositions charges-pigments sont obtenues par mélange physique de la charge minérale avec le colorant.

Toutefois, un tel procédé de préparation présente un certain nombre d'inconvénients.

Un premier inconvénient réside dans le fait qu'il est difficile, pour une charge minérale donnée, d'obtenir toutes les couleurs souhaitées.

Un autre inconvénient se situe au niveau de la compatibilité du colorant avec le matériau qui va recevoir le pigment. En conséquence, pour un matériau donné, en fonction de cette compatibilité, il ne sera possible que d'utiliser un nombre limité de colorants. Dans le même ordre d'idée, il faudra aussi tenir compte de la dispersibilité du colorant dans ce même matériau.

Par ailleurs, les compositions colorées ainsi obtenues peuvent présenter une résistance faible au niveau de la dégradation photochimique et chimique ainsi que vis à vis de l'abrasion.

L'objet principal de l'invention est de procurer des pigments qui, pour une charge minérale donnée peuvent présenter toute la gamme souhaitable de couleurs, qui soient compatibles avec un grand nombre de matériaux et qui présentent enfin un résistance mécanique et chimique améliorée.

Il est connu selon EP—A 0260952 de préparer un pigment protégé par inclusion du pigment dans du silicate de zirconium en faisant une co-précipitation du pigment choisi, de l'oxyde de zirconium et de la silice.

Par ailleurs, il est décrit dans GB—A 772800 des pigments améliorés contenant des colorants organiques et un gel de silice conduisant ainsi à une poudre finement divisée, stable.

Selon le document GB—A 2117783, on prépare une composition pour le couchage du papier en dispersant un pigment organique coloré, du kaolin et un latex.

Dans ce but, les pigments colorés selon l'invention sont caractérisés en ce qu'ils comprennent:
un oxyde minéral,
au moins un colorant,
le ou les colorants étant au moins en partie inclus dans l'oxyde minéral.

On notera ici que pour l'ensemble de la description, le terme oxyde est utilisé par commodité mais qu'il s'applique aussi aux oxydes hydratés de formule $MOx(H_2O)y$.

Selon une variante particulière de l'invention, l'oxyde minéral précité est choisi dans le groupe comprenant les oxydes ou oxydes hydratés de titane, d'aluminium, de zirconium ou la silice.

Selon l'invention, le procédé de préparation des pigments colorés tels que décrits ci-dessus est caractérisé en ce qu'il comprend les étapes suivantes:
on forme un milieu réactionnel dans lequel on met en présence un précurseur de l'oxyde minéral et au moins un colorant,
on coprécipite ou cristallise l'oxyde et le colorant,
on sépare le pigment formé et la phase liquide du milieu réactionnel.

D'autres caractéristiques et avantages de l'invention seront mieux compris à la lecture de la description et des exemples concrets et non limitatifs qui vont suivre.

La caractéristique essentiellement nouvelle des pigments colorés de l'invention réside dans leur structure, cette structure étant liée à la nature même du procédé de préparation des pigments.

Selon cette structure, le ou les colorants sont en totalité ou en partie inclus dans la masse même de l'oxyde minéral. En d'autres termes, ils sont au moins en partie pris dans la maille constituant l'oxyde.

Grâce à cette caractéristique structurelle, les pigments de l'invention présentent des avantages certains.

Tout d'abord, les colorants peuvent masquer totalement la couleur de l'oxyde minéral de sorte qu'il est ainsi possible d'obtenir des pigments dans toute la gamme de coloration possible.

En outre, comme les colorants sont au moins en partie inclus dans la masse de l'oxyde, le problème des compatibilités colorants-matériaux à traiter est ainsi résolu.

Enfin, pour la même raison que précédemment le colorant étant au moins en partie protégé par une couche d'oxyde, le pigment sera plus résistant notamment face à l'action chimique des solvants lors des lavages.

Leur résistance à l'abrasion permettra aux pigments de l'invention de résister aux forces de cisaillement qui se créent lors de leur mise en oeuvre par exemple lors des malaxages, extrusions ou agitations.

Les différents constituants des pigments de l'invention vont maintenant être décrits.

Ces pigments comprennent tout d'abord un oxyde minéral. A ce titre, on pourra utiliser tout oxyde susceptible d'être utilisé comme charge et d'être obtenu par précipitation ou cristallisation.

On peut citer tout particulièrement la silice, le dioxyde de titane, l'alumine et l'oxyde de zirconium.

2

Tout type de colorant peut être utilisé. Bien entendu, des colorants pourront être employés seuls ou en combinaison. Le choix se fera selon les critères conus de l'homme de l'art en fonction du résultat recherché. Une liste non limitative de ces colorants va être donnée ci-dessous.

Ils ont été regroupés par famille. Chaque paragraphe comporte, pour une famille donnée, le nom du colorant et son numéro dans le Color Index (CI).

Etant entendu que l'invention peut être mise en oeuvre en utilisant un ou plusieurs colorants, on notera que pour toute la suite de la description, ce qui est mentionné en faisant référence à un seul colorant doit s'entendre comme valable également pour plusieurs colorants.

Colorants

| No. C.I. | CI nom générique |
|---|---|
| **Anthraquinone** | |
| 65 300 | Pigment Red 177 |
| — | Pigment Yellow 147 |
| 60 010 | Pigment Violet 31 |
| 60 505 | Solvent Red 111 |
| 61 110 | Solvent Blue 68 |
| 58 840 | Solvent Yellow 163 |
| — | Solvent Blue 132 |
| — | Solvent Blue 122 |
| — | Acid Blue 183 |
| — | Solvent Blue 225 |
| **Dioxazine** | |
| — | Pigment Violet 37 |
| **Flaranthrone** | |
| 70 600 | Pigment Yellow 24 |
| **Indanthrone** | |
| 69 800 | Pigment Blue 60 |
| **Quinacridone** | |
| 73 900 | Pigment Violet 19 |
| | Pigment Red 202 |
| | Pigment Red 207 |
| **Azo condensation** | |
| | Pigment Yellow 128 |
| | Pigment Yellow 93 |
| | Pigment Yellow 94 |
| | Pigment Yellow 95 |
| | Pigment Orange 31 |
| | Pigment Brown 23 |
| | Pigment Red 166 |
| | Pigment Red 220 |
| | Pigment Red 144 |
| | Pigment Red 248 |
| | Pigment Red 221 |
| **cuivre—phthalocyamine alpha** | |
| 74 160 | Pigment Blue 15 |
| 74 160 | Pigment Blue 15:1 |
| 74 160 | Pigment Blue 15:2 |
| **Cuivre phthalocyamine beta** | |
| 74 160 | Pigment Blue 15:3 |
| 74 160 | Pigment Blue 15:4 |
| **Cuivre phthalocyamine halogénée** | |
| 74 260 | Pigment Green 7 |
| 74 265 | Pigment Green 36 |

EP 0 266 248 B1

Colorants

| | No. CI | CI nom générique |
|---|---|---|
| **Isoindolinone** | | |
| Orange 26 | — | Pigment Orange 61 |
| | — | Pigment Yellow 109 |
| | 56 280 | Pigment Yellow 110 |
| **Azométhine** | | |
| Complexe du cuivre | — | Pigment Yellow 129 |
| Complexe du nickel | — | Pigment Orange 65 |
| **Perylene** | | |
| | 71 217 | Pigment Red 224 |
| **Arylamide** | | |
| Arylamide 106 | 11 710 | Pigment Yellow 3 |
| Arylamide 6 | 11 680 | Pigment Yellow 1 |
| Arylamide | 11 741 | Pigment Yellow 74 |
| **Diarylide** | | |
| Diarylide anilide | 21 090 | Pigment Yellow 12 |
| Diarylide m-xylidiole | 21 100 | Pigment Yellow 13 |
| Diarylide o-toluidiole | 21 095 | Pigment Yellow 14 |
| Diarylide p-toluidiole | 21 096 | Pigment Yellow 55 |
| Diarylide o-anisidiole | 21 105 | Pigment Yellow 17 |
| Diarylide dimethoxy chloranilide | 21 108 | Pigment Yellow |
| Diarylide pyrazolone | 21 110 | Pigment Orange 13 |
| Diarylide pyrazolone | 21 115 | Pigment Orange 34 |
| Diarylide pyrazolone | 21 120 | Pigment Red 38 |
| **Colorants azo** | | |
| Azo (Ca) | 13 880 | Pigment Yellow 61 |
| | 13 940 | Pigment Yellow 62:1 |
| Azo (Ba) | | |
| | 15 602 | Pigment Red 46 |
| Azo 2 B (Ca) | 15 865:2 | Pigment Red 48:2 |
| Azo 2 B (Ba) | 15 865:1 | Pigment Red 48:1 |
| Azo 2 B toner (Sr) | 15 865:3 | Pigment Red 48:3 |
| Azo 2 B toner (Mg) | 15 865:5 | Pigment Red 48:5 |
| Azo 2 B toner (Mn) | 15 865:4 | Pigment Red 48:4 |
| Azo 4 B toner (Ca) | 15 850:1 | Pigment Red 57:1 |
| Azo (Mn) | 15 825:4 | Pigment Red 58:4 |
| Azo | 11 765 | Pigment Yellow 49 |
| | 12 470 | Pigment Orange 22 |
| | — | Pigment Red 222 |
| **Dinitralinine orange** | 12 075 | Pigment Orange 5 |
| **Naphtol** | | |
| Naphtol rouge | 12 085 | Pigment Red 4 |
| Naphtol AS | 12 310 | Pigment Red 2 |
| Naphtol AS | 12 370 | Pigment Red 112 |
| Naphtol AS | 12 355 | Pigment Red 23 |
| Naphtol AS | 12 385 | Pigment Red 12 |
| Naphtol AS | 12 420 | Pigment Red 7 |
| Naphtol AS | 12 490 | Pigment Red 5 |
| **Toluidine** | | |
| Toluidine rouge | 12 120 | Pigment Red 3 |
| BON (Mn) | 15 880:2 | Pigment Red 63:2 |
| BON (Mn) | 15 860:2 | Pigment Red 52:2 |
| Lake Red C (Ba) | 15 585:1 | Pigment Red 53:1 |

4

# EP 0 266 248 B1

Colorants

| | No. C.I. | C.I. nom générique |
|---|---|---|
| Basic dye toner | 45 160:3 | Pigment Red 81:1 |
| | 45 160:2 | Pigment Red 169 |
| | 42 535:2 | Pigment Violet 3 |
| | 42 535:3 | Pigment Violet 27 |
| | 42 595:2 | Pigment Blue 1 |
| | — | Pigment blue 62 |
| | — | Pigment Green 45 |
| Complexe du fer (Na) | 10 006 | Pigment Green 8 |
| (sulfo) chromate de plomb | 77 600/ | |
| | 77 603 | Pigment Yellow 34 |
| Chromate de plomb | 77 600 | Pigment Yellow 34 |
| Mélange sulfo chromate de plomb-molybdate | 77 605 | Pigment Red 104 |
| Monoazo sans métal | — | Solvant Yellow 146 |
| Complexes du chrome | — | Acid Yellow 118 |
| Complexes du chrome | — | Acid Orange 88 |
| Complexes du chrome | — | Acid Brown 21 |
| Complexes du chrome | — | Acid Red 211 |
| Complexes du chrome | — | Acid Black 172 |
| Complexes du chrome | 18 690 | Solvent Yellow 21 |
| Complexes du chrome | — | Solvent Red 213 |
| Complexes du chrome | — | Solvent Red 7 |
| Complexes du chrome | — | Solvent Red 214 |
| Complexes du chrome | — | Solvent Yellow 88 |
| Complexes du chrome | — | Solvent Orange 59 |
| Complexes du chrome | — | Solvent Red 130 |
| Complexes du cobalt | — | Solvent Yellow 79 |
| Complexes du cobalt | — | Solvent Yellow 25 |
| Complexes du cobalt | — | Solvent Orange 11 |
| Complexes du cobalt | — | Solvent Red 125 |
| Complexes du cobalt | — | Solvent Violet 24 |

On peut aussi mentionner les complexes du nickel et les complexes aminés de cuivre, du nickel et du cobalt.

En ce qui concerne la quantité de colorants présents dans les pigments de l'invention, celle-ci peut varier dans de larges proportions. Généralement, le rapport en poids colorant/oxyde minéral est au plus égal à 10%. Bien entendu, il n'y aurait aucun empêchement à utiliser une quantité plus grande. Cela n'aurait en fait que peu d'intérêt car il apparaît que, grâce au procédé de l'invention, on a généralement besoin pour obtenir des couleurs identiques de l'ordre de dix fois moins de colorants que dans le cas des procédés classiques par mélange. Notamment, le rapport en poids colorant/oxyde minéral sera généralement compris entre 0,001 et 10%.

Enfin, d'une manière générale, on notera que l'on utilise de préférence les colorants cationiques ou basiques dans le cas des pigments à base de silice, les colorants acides ou anioniques pour ceux à base d'alumine.

On préfère aussi utiliser les colorants hydrosolubles lorsque les procédés de préparation sont mis en oeuvre en milieu aqueux. Pour ceux en milieu alcool, on emploie de préférence les colorants solubles dans l'alcool.

Le procédé de préparation des produits selon l'invention va maintenant être décrit plus précisément.

Le principe de base de ce procédé consiste à former l'oxyde minéral en présence du ou des colorants choisis. Il va de soi que l'on peut utiliser tout procédé connu de précipitation et de cristallisation, notamment pour la préparation de silice, d'alumine, d'oxyde de titane ou d'oxyde de zirconium dans la mesure où cette préparation peut se faire en présence d'un colorant. C'est pourquoi, les procédés qui vont être développés ci-dessous ne sont donnés qu'à titre d'exemples, l'homme de l'art pouvant naturellement y substituier d'autres méthodes.

Dans le cas de la préparation d'un pigment coloré à base de silice, on peut citer en particulier deux modes de réalisation.

Le premier mode consiste à former le milieu réactionnel en mettant en présence le colorant, un silicate et un acide. Dans le cas de ce premier mode, deux variantes sont possibles. Selon la première variante, la silice est préparée par addition simultanée de l'acide et d'une solution aqueuse de silicate. Le colorant peut être présent en pied de cuve avant l'introduction simultanée des réactifs ou bien, celui-ci peut être amené

5

simultanément avec ces même réactifs. Dans ce dernier cas, le colorant est amené soit séparément, soit sous forme d'une solution dans le silicate.

Selon un mode de réalisation particulier de cette première variante, on peut introduire l'acide et le silicate en maintenant constant le pH du milieu réactionnel. Généralement, ce pH est fixé à une valeur comprise entre 8 et 9.

La température de réaction peut varier largement. En général, cette température est comprise entre 60 et 100°C.

Une fois la réaction terminée, il est possible d'abaisser le pH du milieu réactionnel par exemple jusqu'à une valeur d'environ 4. On peut aussi effectuer un mûrissement sur une durée variant entre 1/2 heure et 1 heure par exemple. L'abaissement du pH en fin de réaction permet de transformer le silicate restant en silice et d'obtenir ainsi une surface des particules qui ne soit pas trop basique.

La deuxième variante consiste à préparer la silice à partir d'un pied de cuve comprenant une solution de silicate. En d'autres termes, on forme tout d'abord une solution aqueuse de silicate, on y introduit le colorant. Le colorant est maintenu à l'état dispersé dans la solution par une agitation convenable. Ensuite, on ajoute un acide.

Dans cette deuxième variante, les conditions de température sont identiques à celles décrites dans la première. Il est bien entendu possible d'effectuer aussi un mûrissement.

La séparation du pigment formé de la phase liquide du milieu réactionnel obtenu selon les procédés décrits se fait ensuite d'une manière connue en soi. Le pigment séparé est ensuite séché.

En ce qui concerne le silicate, on utilise généralement des silicates alcalins et plus particulièrement les silicates de sodium, de potassium ou de lithium. D'une manière connue, on emploie des silicates de rapport molaire généralement compris entre 2 et 4.

Pour les acides, on utilise habituellement un acide choisi dans le groupe comprenant les acides sulfurique, nitrique, chlorhydrique, carbonique.

Un deuxième mode de réalisation va maintenant être décrit.

Ce mode consiste essentiellement à préparer la silice par hydrolyse d'un alkyl-silicate.

Plus précisément, on met en présence au moins un colorant et un alkyl-silicate, on hydrolyse cet alkyl-silicate et on sépare le pigment formé et la phase liquide du milieu réactionnel.

Il s'agit là de la voie plus particulièrement décrite dans l'article de Stöber et al. Journal of Colloid and Interface Science 26 62—69 (1968) dont l'enseignement est incorporé à la présente description.

Généralement, il est préférable d'effectuer l'hydrolyse en présence d'une base qui joue le rôle de catalyseur.

On procède en formant un milieu réactionnel par mélange d'eau, d'alcool et, éventuellement d'une base, en introduisant ensuite l'alkyl-silicate, le ou les colorants étant, soit introduits simultanément, soit déjà présents dans le milieu réactionnel avant l'introduction de l'alkyl-silicate.

On peut utiliser comme base l'ammoniaque. Les alcools utilisés sont généralement des alcools aliphatiques. La réaction a habituellement lieu à température ambiante. L'alkyl-silicate est de préférence introduit avec un alcool.

Il est aussi possible de faire un pied de cuve à base d'alcool, de colorant, d'alkyl-silicate et d'y introduire ensuite l'eau ou un mélange eau-base.

Comme alkyl-silicate, on utilise en particulier le silicate d'éthyle.

Comme précédemment, le pigment obtenu est séparé du milieu réactionnel puis lavé en général à l'alcool, puis séché.

Dans le cadre de la préparation de pigments colorés à base d'alumine plusieurs mode de réalisation sont concevables.

Selon un premier mode de réalisation, on forme le milieu réactionnel en mettant en présence au moins un colorant, un aluminate et un acide. Plusieurs variantes sont possibles. Ainsi, on pourra introduire simultanément l'aluminate et l'acide, le colorant étant présent en pied de cuve avant l'introduction simultanée ou bien, dans un autre cas, le colorant étant amené simultanément.

On peut opérer de manière à maintenir constant le pH du milieu réactionnel.

Selon une autre variante, on peut partir d'une solution d'aluminate. On y introduit le colorant et on y ajoute simlutanément ou postérieurement l'acide.

On utilise généralement un aluminate alcalin. L'acide utilisé peut être par exemple l'acide chlorhydrique ou nitrique.

Selon un deuxième mode de réalisation la préparation d'un pigment à base d'alumine se fait en formant un milieu réactionnel en mettant en présence au moins un colorant, un sel d'aluminium et une base.

Bien entendu, les variantes qui ont été décrites pour le mode de réalisation précédent s'appliquent tout aussi bien à celui-ci. Ainsi, on pourra partir d'un pied de cuve constitué par une solution du sel d'aluminium, avec introduction simultanée ou séparée du colorant et de la base.

La base est généralement la soude ou l'ammoniaque, le sel d'aluminium pouvant être par exemple un halogénure d'aluminium tel que le chlorure d'aluminium ou un nitrate d'aluminium.

Cette variante est à rapprocher de celle écrite plus haut pour l'hydrolyse d'un alkyl-silicate.

On procède en mettant en présence au moins un colorant et un alcoolate d'aluminium, on hydrolyse cet alcoolate et on sépare le pigment formé et la phase liquide du milieu réactionnel.

Tout ce qui a été décrit plus haut dans le cas de l'hydrolyse d'un alkyl-silicate s'applique ici notamment en ce qui concerne l'emploi d'une base et le mode d'introduction des réactifs.

On peut utiliser à titre d'alcoolate le méthylate, l'éthylate, l'isopropylate, le butylate d'aluminium, ces alcoolates étant sous forme liquide ou solide en dispersion ou en solution dans un solvant organique, par exemple le benzène ou l'alcool correspondant.

Dans le cas de la préparation de pigments colorés à base de dioxyde de titane, celui-ci peut être préparé en présence du ou des colorants selon différentes méthodes.

La première consiste à mettre en présence le support, au moins un colorant et un sel de titane IV, à hydrolyser ce sel et à séparer le pigment ainsi formé et la phase liquide du milieu réactionnel.

Plus précisément, cette hydrolyse pourra se faire sur des solutions sulfuriques de sulfate de titane IV. Ces solutions après évaporation, en vue d'obtenir une solution concentrée, sont mélangées avec de l'eau chaude à température d'environ 95°C. L'ensemble est maintenu en ébullition. On recueille ainsi un précipité.

Dans un tel cas, le colorant est présent au départ dans la solution de sulfate de titane IV.

Une autre possibilité consiste à réaliser une hydrolyse du chlorure de $TiCl_4$ avec addition d'ammoniaque. Le colorant peut être présent dans la solution de départ de $TiCl_4$.

Une troisième voie consiste à effectuer une hydrolyse d'un titanate d'alkyle. Cette troisième voie est identique à celles qui ont été décrites plus haut concernant l'hydrolyse d'un alkyl-silicate ou d'un alcoolate d'aluminium. Tout ce qui a été décrit plus haut à ce sujet concernant notamment le mode d'introduction des réactifs s'applique ici.

Dans le cadre de la préparation de pigments colorés comprenant l'oxyde de zirconium comme oxyde minéral, les méthodes de préparation sont du même type que celles qui viennent d'être décrites plus haut pour le dioxyde de titane.

Les pigments de l'invention peuvent avoir de nombreuses applications dans tous les domaines mettant en oeuvre des charges colorées. On pourra les utiliser par exemple dans la préparation de cosmétiques, de compositions détergentes, de colles.

Outre leur résistance chimique les pigments de l'invention présentent une grande dispersibilité.

C'est ainsi qu'ils peuvent entrer dans la préparation de compositions dentifrices. En effet les colorants utilisés habituellement dans ces compositions sont difficiles à disperser dans la pâte dentifrice et il est nécessaire d'ajouter alors des tensio-actifs. Par ailleurs la couleur se maintient mal au cours du temps.

Dans le cas des pigments selon l'invention leur incorporation dans les dentifrices se fait facilement à cause justement de leur bonne dispersibilité. En outre, on observe qu'il n'y a pas de variation de la coloration du dentifrice au cours du temps. Les colorants utilisés à cet effet seront par exemple ceux du type chlorophylline, bleu de thymol, fluoresceine (CI 45350), les pigments blanc (CI 74160) et vert (CI 74260).

Ces pigments peuvent aussi être utilisés avantageusement dans la préparation de poudres de développement de type électrostatique pour les procédés électrographiques ou xérographiques.

Cette utilisation des pigments de l'invention n'entraîne pas de modification en ce qui concerne la formulation des poudres précitées. C'et ainsi qu'outre le pigment, elles pourront comporter tous les autres ingrédients usuels, tels que les liants et autres additifs comme par exemple les agents de charges.

Des exemples concrets mais non limitatifs de l'invention vont maintenant être donnés.

Exemple 1—Préparation de silice sphérique colorée

Dans un réacteur inox de 4 litres équipé d'un agitateur type Mixel et contre-pâles, on introduit les réactifs suivants constituant le pied de cuve.

| | |
|---|---|
| 1—Alcool 96° | 2 000 ml |
| 2—NH$_4$OH (à 240 g/l) | 398 ml |
| 4—H$_2$O permutée | 114 ml |
| 5—Colorant rouge | |
| Bayplast de Bayer | 1 g |

A température ambiante, on introduit un mélange de 775 ml de silicate d'éthyl $Si(OEH_2)_4$ et 213 ml d'éthanol à la vitesse de 25 cm³/min. La suspension colorée est ensuite filtrée sur buchner, le filtrat est lavé à l'alcool à 90°C séché à l'air 15 heures puis à 120°C pendant 4 heures.

Diamètre moyen des particules de silice: 0,75 µm.

Exemple 2—Préparation de silice colorée

Dans un réacteur inox de 3 litres équipé d'un agitateur type turbine 4 pâles Ø 45 mm. On introduit en premier un mélange de:

—500 cm³ silicate de sodium de rapport molaire Rm SiO$_2$/Na$_2$O de 3,4 380 g/l en SiO$_2$
—1000 cm³ H$_2$O permutée.

La solution agitée à 400 tr/min est portée à 80°C et maintenue à cette température par régulation, on disperse alors progressivement 2 g de colorant Irgalithe jaune 133 R de Ciba-Geigy. A l'aide d'une pompe doseuse au débit de 45,6 cm³/min on ajoute 930 cm³ d'$H_2SO_4$ 9% en 20 minutes. Le pH de 10,6 au départ tombe à 8,6 en fin d'introduction.

Par une addition supplémentaire de 250 cm³ d'$H_2SO_4$ 9% on ajuste le pH à 7,5.

Le mélange est filtré sur buchner et filtré sur papier, puis lavé par 3 fois son volume d'eua permutée.

Le produit est séché en étuve à 120°C durant 15 heures.

### Exemple 3—Préparation d'oxyde de titane coloré

On part d'un pied de cuve comprenant 250 ml de butanol et un colorant rouge Irgalithe de Ciba-Geigy. A 60°C sous agitation par turbine à 200 tr/min, on ajoute simultanément 1 l de Ti(OBu)₄ à 68 g/l dans le butanol-1 et 1 l d'$H_2O$ à 14,4 g/l dans le butanol-1 pendant 1 heure. On filtre, on lave et sèche.

### Exemple 4—Préparation d'alumine coloré

Dans un réacteur de 3 l, on introduit sous agitation par une turbine à 800 tr/min, 2 l d'une solution aqueuse contenant 5,5 g de $Na_2SO_4$ et 0,1 g de colorant rouge Irgalithe de Ciba.

On procède par la suite à une addition de 15 cm³ de butanol-2 contenant 5 g de butylate d'aluminium (Al(OBu)3) en 30 mm.

Le mélange est ensuite porté à 95°C en 2 heures, puis est laissé murir pendant 20 heures.

Ensuite, le mélange est filtré sur filtre Millipore 0,8 μm et lavé à l'eau permutée.

Le produit est séché en étuve à 130°C pendant 15 h.

Après broyage, il présente une coloration rouge.

### Exemple 5—Préparation d'alumine colorée

Dans un réacteur de 1 litre, on introduit, sous agitation par une turbine 4 pales à 800 tr/min 200 cm³ d'$H_2O$ permutée et 1 g de Bleu mordant 3 (Color Index No. 43 820).

Le mélange est porté à 70°C.

On procède par la suite à une addition simultanée d'une solution d'aluminate de sodium (100 g d'$Al_2O_3$ dans 300 cm³ d'$H_2O$) avec un débit constant de 10 cm³/min et d'$H_2SO_4$ à 5%. Le débit d'$H_2SO_4$ étant ajusté pour maintenir le pH à une valeur constante de 8.

Le mélange est ensuite laissé murir pendant 1 heure. Après ce temps, on abaisse le pH par $H_2SO_4$ à 5%.

Ensuite, le mélange est filtré sur Buchner.

Le lavage est effectué à l'eau permutée.

Le produit est séché en étuve à 130°C pendant 15 heures.

Après broyage, il présente une coloration bleue.

### Exemple 6

Cet exemple a pour but de montrer la tenue à la coloration aux solvants des pigments colorés selon l'invention.

On utilise un tube de 50 cm³ bouché hermétiquement dans lequel on a placé 1 g de pigment à tester des exemples 2 et 5 et 20 g d solvant qui peut être l'eau $H_2O$, le méthanol (MeOH), l'heptane (C₇).

On homogénéise le milieu par passage du tube dans un agitateur Turbula pendant 2 heures.

On observe ensuite la sédimentation pendant 1 heure, 24 heures et 48 heures.

Les résultats sont donnés dans le tableau ci-dessous.

On peut voir que les colorants des pigments selon l'invention ne sont pas éliminés par lavage à l'eau, au méthanol et à l'heptane.

La coloration trouble de l'exemple 2 est due à la sédimentation lente du pigment dans le solvant.

| Pigment | Solvant | Temps en heures | | |
| | | 1 | 24 | 48 |
| --- | --- | --- | --- | --- |
| Exemple 2 | $H_2O$ | Incolore | | |
| | MeOh | Coloré | Coloré | Incolore |
| | | Trouble | Trouble | |
| | C₇ | Incolore | | |
| Exemple 5 | $H_2O$ | Incolore | | |
| | MeOH | Incolore | | |
| | C₇ | Incolore | | |

### Exemple 7—Préparation de silice colorée pour dentifrice

On introduit dans un réacteur 6,00 l d'eau déionisée. On y ajoute, sous agitation, 10,2 g de chlorophylline en poudre hydrosoluble.

On chauffe sous agitation jusqu'à 85°C.

Lorsque cette température est atteinte, on introduit simultanément dans le réacteur 8,5 l de silicate de sodium (d=1,112) à un débit de 0,340 l/mn et 13,5 l d'acide sulfurique (80,0 g/l) à un débit de 0,210 l/mn.

Le débit d'acide sulfurique est ajusté de manière à maintenir le pH du milieu à une valeur constante de 8.

On effectue l'addition simultanée pendant 40 mn.

Le pH moyen au cours de la réaction est de 8,0.

A la fin de l'addition simultanée on laisse mûrir le mélange réactionnel pendant 10 mn à pH 8.

Le pH est ensuite stabilisé à la valeur 5 par ajout d'acide sulfurique.

On réalise un mûrissement de 15 mn à pH 5,0 et à 85°C.

La bouillie est ensuite filtrée et le gâteau obtenu est lavé à l'eau désionisée.

On sèche la silice dans une étuve à 120°C pendant 6 heures.

La silice obtenue est ensuite broyée pour obtenir un diamètre moyen des particules de 3 microns.

La silice obtenue est caractérisée par une surface BET de 70 m²/g et une couleur verte permettant d'obtenir des pâtes dentifrices vertes.

Exemple 8

Dans un réacteur équipé d'un système de régulation de température et de pH et d'un système d'agitation par turbine, on introduit 6 l d'eau déionisée et 100 g de pigment bleu (CI 74180).

Après la mise en marche de l'agitation (300 t/mn), le pied de cuve ainsi constitué est chauffé à 85°C.

Lorsque la température est atteinte, on procède à l'addition simultanée de 8,5 l de silicate de sodium de concentration en silice de 120 g/l, de rapport $SiO_2/Na_2O$ égal à 3,5 et de débit 0,34 l/mn et de 13,5 l d'acide sulfurique de concentration 80 g/l. Le débit d'acide est ajusté de manière à maintenir le pH du milieu à une valeur constante de 8,0.

Après 40 mn d'addition, on arrête l'addition de silicate et on continue l'addition d'acide jusqu'à stabiliser le pH du mélange réactionnel à 4.

On réalise par la suite un mûrissement de 15 mn à ce pH et à 85°C.

Le mélange est ensuite filtré et le gâteau humide est lavé à l'eau déionisée.

Le produit est ensuite séché par atomisation et broyé sur un broyeur de type forplex pour obtenir une granulométrie de 10 microns. La silice présente une surface BET de 60 m²/g et une coloration bleue.

On utilise ensuite la formulation dentifrice ci-dessous (les pourcentages sont en poids):

| | |
|---|---|
| Glycérine | 22% |
| Carboxyméthylcellulose (7 mFD) | 1% |
| Silice colorée bleue | 31% |
| Benzoate de sodium | 0,1% |
| Saccharinate de sodium | 0,2% |
| Fluorure de sodium | 0,1% |
| Arôme | 0,9% |
| Eau | 37% |

Le dentifrice obtenu est facilement extrudable, il présente une bonne tenue sur la brosse.

L'utilisation de silice colorée facilite la formulation, la dispersibilité est parfaite, très rapide et ne nécessite pas l'addition préalable de tensio-actifs.

De plus aucun effet de vieillissement n'a été constaté après 6 mois à 120°C.

Exemple 9

Cet exemple décrit la préparation d'une silice pour poudre de développement.

Dans un réacteur de 3 litres équipé d'un système d'agitation (turbine), de régulation de température et de pH, on introduit 1000 ml d'eau et 500 ml de silicate de sodium de concentration en silice de 380 g/l et de Rm=3,5.

Sous agitation et à 80°C on disperse 2 g de pigment Irgalithe 2 GP (Ciba). On procède par la suite à l'addition de 900 ml d'$H_2SO_4$ (10%) en 40 minutes.

Après un mûrissement de 15 mn, on descend le pH à 5 par addition d'$H_2SO_4$ (10%).

Le mélange est filtré et lavé puis séché sur un microniseur type Jet Pulverizer.

Exemple 10

Le même protocole permet d'obtenir des pigments de différentes couleurs à partir des colorants ci-dessous:

| | |
|---|---|
| Jaune: | Microlithe 2GT (Ciba) |
| Jaune: | Irgalithe 2GP (Ciba) |
| Magenta: | Rubine Irgalithe LPBC (Ciba) |
| Magenta: | Rubis lithiol (BASF) |
| Magenta: | Isol 4 BK (KVK) |
| Cyan: | Bleu héliogène D 7072 (BASF) |
| Cyan: | Isol G4B (KVK). |

Exemple 11

La transparence des pigments étant une qualité recherchée pour leur application à la préparation de poudres de développement, cet exemple est relatif à des tests de mesure de la lumière transmise par un film.

Une partie en poids de pigment est dispersée dans une solution constituée de 10 parties de résine polyester (type Norsodyne de CDF) et 100 parties de tétrahydrofuranne.

La dispersion est déposée sous forme de couche mince d'épaisseur 3 µm sur un film polyester de 50 µm.

(inverse du logarithme de la transmittance).

La densité de lumière transmise (inverse du logarithme de la transmittance) est mesurée à l'aide d'un densitomètre.

Ci-dessous, on compare les densités de lumière transmise de deux enductions obtenues selon le porotocole décrit ci-dessus.

L'enduction A est obtenue à partir de pigment pur (Ergalithe 2GP) et l'enduction B est obtenue à partir de pigment de silice colorée décrit dans l'exemple 9.

| Epaisseur de la couche mince (µm) | Densité de lumière transmise | |
|---|---|---|
| | Pigment A | Pigment B |
| 3 | 0,60 | 0,10 |

Il apparaît que les pigments de silice colorée sont plus transparents.

Bien entendu, l'invention n'est nullement limitée aux modes de réalisation décrits qui n'ont été donnés qu'à titre d'exemples. En particulier, elle comprend tous les moyens constituant des équivalents techniques des moyens décrits ainsi que leurs combinaisons si celles-ci sont mises en oeuvre dans le cadre de la protection comme revendiquée.

**Revendications**

1. Pigment coloré caractérisé en ce qu'il est constitué:
d'un oxyde minéral,
d'au moins un colorant,
le ou les colorants étant au moins en partie inclus dans l'oxyde minéral.

2. Pigment selon la revendication 1, caractérisé en ce que l'oxyde minéral précité est choisi dans le groupe comprenant les oxydes ou oxydes hydratés de titane, d'aluminium, de zirconium et la silice.

3. Pigment coloré, caractérisé en ce qu'il est préparé par un procédé dans lequel, dans une première étape, on forme un milieu réactionnel dans lequel on met en présence un précurseur d'un oxyde minéral et au moins un colorant, dans une deuxième étape, on coprécipite l'oxyde et le colorant et, dans une troisième étape, on sépare le pigment formé et la phase liquide du milieu réactionnel.

4. Pigment selon la revendication 3, caractérisé en ce que l'oxyde minéral est choisi dans le groupe comprenant la silice, le dioxyde de titane, l'alumine, l'oxyde de zirconium.

5. Procédé de préparation d'un pigment coloré du type de l'une des revendications 1 à 4, caractérisé en ce qu'il comprend les étapes suivantes:
on forme un milieu réactionnel dans lequel on met en présence un précurseur de l'oxyde minéral et au moins un colorant,
on coprécipite ou cristallise l'oxyde et le colorant,
on sépare le pigment formé et la phase liquide du milieu réactionnel.

6. Procédé de préparation d'un pigment coloré à base de silice selon la revendication 5, caractérisé en ce que l'on forme le milieu réactionnel en mettant en présence au moins un colorant, un silicate et un acide.

7. Procédé de préparation d'un pigment coloré à base d'alumine selon la revendication 5, caractérisé en ce qu'on forme le milieu réactionnel en mettant en présence au moins un colorant, un aluminate et un acide.

8. Procédé de préparation d'un pigment coloré à base d'alumine selon la revendication 5, caractérisé en ce qu'on forme le milieu réactionnel en mettant en présence au moins un colorant, un sel d'aluminium et une base.

9. Procédé selon l'une des revendications 6 à 8, caractérisé en ce qu'on forme le milieu réactionnel en introduisant simultanément l'acide ou la base d'une part, le silicate ou l'aluminate d'autre part, le colorant étant présent avant l'introduction simultanée, soit amené en cours de réaction, séparément ou sous forme d'une solution dans le silicate, le sel d'aluminium ou l'aluminate.

10. Procédé selon la revendication 9, caractérisé en ce que l'on introduit l'acide ou la base, le silicate ou l'aluminate ou le sel d'aluminium, en maintenant constant le pH du milieu réactionnel.

11. Procédé selon l'une des revendications 6 à 8, caractérisé en ce que l'on forme tout d'abord une solution aqueuse de silicate, d'aluminate ou de sel d'aluminium, on introduit le colorant dans cette solution et on ajoute enfin l'acide ou la base.

# EP 0 266 248 B1

12. Procédé selon l'une quelconque des revendications 5 à 11, caractérisé en ce que après la fin de la réaction on fait mûrir le pigment formé.

13. Procédé selon l'une quelconque des revendications 6 à 12, caractérisé en ce qu'on utilise un silicate ou un alumiante alcalin ou un halogénure ou nitrate d'aluminium.

14. Procédé selon l'une quelconque des revendications 6 à 12, caractérisé en ce qu'on utilise un acide choisi dans le groupe comprenant les acides sulfurique, nitrique, chlohydrique, carbonique.

15. Procédé de préparation d'un pigment coloré à base de silice selon la revendication 5, caractérisé en ce qu'on met en présence un alkyl-silicate et au moins un colorant, on hydrolyse l'alkyl-silicate et on sépare le pigment formé et la phase liquide du milieu réactionnel.

16. Procédé de préparation d'un pigment coloré à base d'alumine selon la revendication 5, caractérisé en ce qu'on met en présence un alcoolate d'aluminium et au moins un colorant, on hydrolyse l'alcoolate et on sépare le pigment formé et la phase liquide du milieu réactionnel.

17. Procédé selon l'une des revedications 15 ou 16, caractérisé en ce que l'hydrolyse a lieu en présence d'une base.

18. Procédé selon l'une des revendications 15 à 17, caractérisé en ce qu'on forme un milieu réactionnel par mélange d'eau, d'alcool et le cas échéant d'une base et en ce qu'on y introduit ensuite l'alkyl-silicate ou l'alcoolate d'aluminium, le colorant étant, soit introduit simultanément, soit présent dans le milieu réactionnel avant l'introduction de l'alkyl-silicate ou de l'alcoolate.

19. Procédé de préparation d'un pigment coloré à base de dioxyde de titane selon la revendication 5, caractérisé en ce que l'on met en présence un sel de titane IV et au moins un colorant, on hydrolyse ce sel et on sépare le pigment formé et la phase liquide du milieu réactionnel.

20. Procédé de préparation d'un pigment coloré à base de dioxyde de titane selon la revendication 5, caractérisé en ce qu'il comporte les étapes suivantes:

on met en présence au moins un colorant et un titanate d'alkyle,

on hydrolyse le titanate d'alkyle,

on sépare le pigment formé et la phase liquide du milieu réactionnel.

21. Procédé selon l'une des revendications 5 à 20, caractérisé en ce que après séparation du milieu réactionnel, le pigment est lavé et séché.

22. Poudre de développement de type électrostatique, caractérisée en ce qu'elle comprend un pigment selon l'une des revendications 1 à 4 ou un pigment préparé par le procédé selon l'une des revendications 5 à 21.

23. Composition dentifrice caractérisée en ce qu'elle comprend un pigment selon l'une des revendications 1 à 4 ou un pigment préparé par le procédé selon l'une des revendications 5 à 21.

## Patentansprüche

1. Gefärbtes Pigment, dadurch gekennzeichnet, daß es besteht aus:
einem Mineraloxid
wenigstens einem Farbstoff
und der oder die Farbstoffe wenigstens teilweise im Mineraloxid eingeschlossen sind.

2. Pigment nach Anspruch 1, dadurch gekennzeichnet, daß das zuvor genannte Mineraloxid aus der Gruppe ausgewählt ist, die die Oxide oder Oxidhydrate des Titans, des Aluminiums, des Zirkoniums und Siliciumdioxid umfaßt.

3. Gefärbtes Pigment, dadurch gekennzeichnet, daß es durch ein Verfahren hergestellt wird, worin man in einer ersten Stufe ein Reaktionsmilieu herstellt, in dem man eine Vorstufe eines Mineraloxids und wenigstens einen Farbstoff zusammenführt, in einer zweiten Stufe das Oxid und den Farbstoff gemeinsam ausfällt und in einer dritten Stufe das gebildete Pigment von der Flüssigphase des Reaktionsmilieus trennt.

4. Pigment nach Anspruch 3, dadurch gekennzeichnet, daß das Mineraloxid aus der Gruppe ausgewählt ist, die Siliciumdioxid, Titandioxid, Aluminiumoxid und Zirkoniumoxid umfaßt.

5. Verfahren zur Herstellung eines gefärbten Pigments des Typs eines der Ansprüche 1 bis 4, dadurch gekennzeichnet, daß es die folgenden Stufen umfaßt:
man stellt ein Reaktionsmilieu her, worin man eine Vorstufe des Mineraloxids und wenigstens einen Farbstoff zusammenführt,
man kristallisiert oder fällt Oxid und Farbstoff gemeinsam aus,
man trennt das gebildete Pigment von der Flüssigphase des Reaktionsmilieus.

6. Verfahren zur Herstellung eines gefärbten Pigmentes auf Siliciumdioxidgrundlage nach Anspruch 5, dadurch gekennzeichnet, daß man das Reaktionsmilieu durch Zusammenführen wenigstens eines Farbstoffes, eines Silicates und einer Säure herstellt.

7. Verfahren zur Herstellung eines gefärbten Pigmentes auf Aluminiumoxidgrundlage nach Anspruch 5, dadurch gekennzeichnet, daß man das Reaktionsmilieu durch Zusammenführen wenigstens eines Farbstoffes, eines Aluminates und einer Säure herstellt.

8. Verfahren zur Herstellung eines gefärbten Pigmentes auf Aluminiumoxidgrundlage nach Anspruch 5, dadurch gekennzeichnet, daß man das Reaktionsmilieu durch Zusammenführen wenigstens eines Farbstoffes, eines Aluminiumsalzes und einer Base herstellt.

9. Verfahren nach einem der Ansprüche 6 bis 8, dadurch gekennzeichnet, daß man das Reaktionsmilieu

# EP 0 266 248 B1

durch gleichzeitiges Einführen von einerseits der Säure oder der Base und andererseits des Silicates oder Aluminates herstellt, wobei der Farbstoff entweder vor der gleichzeitigen Zugabe vorhanden ist oder im Reaktionsverlauf allein oder in Form einer Lösung in das Silicat, das Aluminiumsalz oder das Aluminat eingeführt wird.

10. Verfahren nach Anspruch 9, dadurch gekennzeichnet, daß man die Säure oder die Base, das Silicat oder das Aluminat oder das Aluminiumsalz bei Konstanthalten des pH-Wertes des Reaktionsmilieus zugibt.

11. Verfahren nach einem der Ansprüche 6 bis 8, dadurch gekennzeichnet, daß man zuerst eine wässrige Lösung des Silicates, Aluminates oder Aluminiumsalzes herstellt, in diese den Farbstoff einbringt und schließlich die Säure oder die Base zufügt.

12. Verfahren nach einem der Ansprüche 5 bis 11, dadurch gekennzeichnet, daß man nach Abschluß der Umsetzung das gebildete Pigment wachsen läßt.

13. Verfahren nach einem der Ansprüche 6 bis 12, dadurch gekennzeichnet, daß man ein Alkalisilicat oder ein -aluminat oder ein Aluminiumhalogenid oder ein -nitrtat verwendet.

14. Verfahren nach einem der Ansprüche 6 bis 12, dadurch gekennzeichnet, daß man eine Säure verwendet, die aus der Gruppe ausgewählt ist, die die Schwefel-, Salpeter-, Salz- und Kohlensäure umfaßt.

15. Verfahren zur Herstellung eines gefärbten Pigmentes auf Siliciumdioxidgrundlage nach Anspruch 5, dadurch gekennzeichnet, daß man ein Alkylsilicat und wenigstens einen Farbstoff zusammenführt, das Alkylsilicat hydrolysiert und das gebildete Pigment von der Flüssigphase des Reaktionsmilieus trennt.

16. Verfahren zur Herstellung eines Farbpigmentes auf Aluminiumoxidgrundlage nach Anspruch 5, dadurch gekennzeichnet, daß man ein Aluminiumalkoholat und wenigstens einen Farbstoff zusammenführt, das Alkoholat hydrolysiert und das gebildete Pigment von der Flüssigphase des Reaktionsmilieus trennt.

17. Verfahren nach einem der Ansprüche 15 oder 16, dadurch gekennzeichnet, daß die Hydrolyse in Gegenwart einer Base stattfindet.

18. Verfahren nach einem der Ansprüche 15 bis 17, dadurch gekennzeichnet, daß man ein Reaktionsmilieu durch Mischen von Wasser, Alkohol und gegebenenfalls einer Base bildet und daß man in dieses anschließend das Alkylsilicat oder das Aluminiumalkoholat einbringt, wobei der Farbstoff entweder gleichzeitig zugegeben wird oder im Reaktionsmilieu vor der Zugabe des Alkylsilicates oder des Alkoholates vorhanden ist.

19. Verfahren zur Herstellung eines gefärbten Pigmentes auf Titandioxidgrundlage nach Anspruch 5, dadurch gekennzeichnet, daß man ein Titan(IV)-Salz und wenigstens einen Farbstoff zusammenführt, dieses Salz hydrolysiert und das gebildete Pigment von der Flüssigphase des Reaktionsmilieus trennt.

20. Verfahren zur Herstellung eines gefärbten Pigmentes auf Titandioxidgrundlage nach Anspruch 5, dadurch gekennzeichnet, daß es die folgenden Stufen umfaßt:
man führt wenigstens einen Farbstoff und ein Alkyltitanat zusammen,
man hydrolysiert das Alkyltitanat,
man trennt das gebildete Pigment von der Flüssigphase des Reaktionsmilieus.

21. Verfahren nach einem der Ansprüche 5 bis 20, dadurch gekennzeichnet, daß das Pigment nach der Trennung vom Reaktionsmilieu gewaschen und getrocknet wird.

22. Entwicklungspulver des elektrostatischen Typs, dadurch gekennzeichnet, daß es ein Pigment nach einem der Ansprüche 1 bis 4, oder ein durch das Verfahren nach einem der Ansprüche 5 bis 21 hergestelltes Pigment umfaßt.

23. Zubereitung zur Zahnreinigung, dadurch gekennzeichnet, daß sie ein Pigment nach einem der Ansprüche 1 bis 4 oder ein durch das Verfahren nach einem der Ansprüche 5 bis 21 hergestelltes Pigment umfaßt.

**Claims**

1. Coloured pigment, characterized in that it is constituted by mineral oxide and at least one dye, the dye or dyes being at least partly included in the mineral oxide.

2. Pigment according to Claim 1, characterized in that the aforementioned mineral oxide is chosen from the group including oxides or hydrated oxides of silica, titanium, aluminium and zirconium.

3. Coloured pigment, characterized in that it is prepared by a process in which, in a first stage, is formed a reaction medium in which are brought together a precursor of a mineral oxide and at least one dye, in a second stage the oxide and dye are coprecipitated and in a third stage the pigment formed and the liquid phase are separated from the reaction medium.

4. Pigment according to Claim 3, characterized in that the mineral oxide is chosen from among silica, titanium dioxide, alumina and zirconium oxide.

5. A process for the preparation of a coloured pigment according to any one of the Claims 1 to 4, characterized in that it comprises the stages of forming a reaction medium in which are placed a precursor of the mineral oxide and at least one dye, the oxide and dye are coprecipitated or crystallized and the pigment formed and the liquid phase are separated from the reaction medium.

6. A process for the preparation of a silica-based coloured pigment according to Claim 5, characterized in that the reaction medium is formed by bringing together at least one dye, a silicate and an acid.

7. Process for the preparation of an alumina-based coloured pigment according to Claim 5, characterized in that the reaction medium is formed by bringing together at least one dye, an aluminate and an acid.

8. Process for the preparation of an alumina-based coloured pigment according to Claim 5, characterized in that the reaction medium is formed by bringing together one dye, an aluminium salt and a base.

9. Process according to any one of the Claims 6 to 8, characterized in that the reaction medium is formed by simultaneously introducing the acid or the base on the one hand, the silicate or the aluminate on the other, the dye being present either prior to the simultaneous introduction, or introduced during the reaction separately or in the form of a solution inside the silicate, the aluminum salt or the aluminate.

10. Process according to Claim 9, characterized in that the acid or base, silicate or aluminate or aluminium salt is introduced by maintaining constant the pH of the reaction medium.

11. Process according to one of the Claims 6 to 8, characterized in that firstly an aqueous solution of silicate, aluminate or aluminium salt is formed, the dye being introduced into said solution and finally the acid or base is added.

12. Process according to any one of the Claims 5 to 11, characterized in that the pigment formed is aged after the end of the reaction.

13. Process according to any one of the Claims 6 to 12, characterized in that use is made of an alkaline aluminate or silicate or an aluminium nitrate or halide.

14. Process according to any one of the Claims 6 to 12, characterized in that use is made of an acid chosen from among sulphuric, nitric, hydrochloric and carboxylic acids.

15. Process for the preparation of a silica-based coloured pigment according to Claim 5, characterized in that an alkyl-silicate and at least one dye brought together, the alkyl-silicate is hydrolyzed and the pigment formed and the liquid phase are separated from the reaction medium.

16. Process for the preparation of an alumina-based coloured pigment according to Claim 5, characterized in that an aluminium alkoxide is brought together with at least one dye, the alkoxide is hydrolyzed and the pigment formed and the liquid phase are separated from the reaction medium.

17. Process according to one of the Claims 15 or 16, characterized in that hydrolysis takes place in the presence of a base.

18. Process according to one of the Claims 15 to 17, characterized in that a reaction medium is formed by mixing water, alcohol and, if appropriate, a base and that into the same is subsequently introduced the alkyl silicate or aluminium alkoxide, the dye being either simultaneously introduced, or present in the reaction medium prior to the introduction of the alkyl silicate or alkoxide.

19. Process for the preparation of a titanium dioxide-based coloured pigment according to Claim 5, characterized in that a titanium (IV) salt amd at least one dye are brought together, the salt is hydrolyzed and the pigment formed and liquid phase are separated from the reaction medium.

20. Process for the preparation of a titanium dioxide-based coloured pigment according to Claim 5, characterized in that it comprises the stages of bringing together at least one dye and an alkyl titanate, the alkyl titanate is hydrolyzed and the pigment formed and liquid phase are separated from the reaction medium.

21. Process according to one of the Claims 5 to 20, characterized in that the pigment is washed and dried after separation from the reaction medium.

22. Electrostatic development powder, characterized in that it comprises a pigment according to any one of the Claims 1 to 4 or a pigment prepared by the process according to one of the Claims 5 to 21.

23. Dentifrice composition, characterized in that it comprises a pigment according to one of the Claims 1 to 4 or a pigment prepared by the process according to one of the Claims 5 to 21.